# EUROPÄISCHE PATENTSCHRIFT

(11) **EP 2 107 047 B1**
(45) Veröffentlichungstag und Bekanntmachung des Hinweises auf die Patenterteilung: **28.09.2011**
(21) Anmeldenummer: 09155811.4
(22) Anmeldetag: 23.03.2009
(51) Int. Cl.: C07B 49/00, C07C 1/32

(54) **Verfahren zur Herstellung organischer Verbindungen durch eine Übergangsmetall-katalysierte Kreuzkupplungsreaktion einer Aryl-X-, Heteroaryl-X-, Cycloalkenyl-X- oder Alkenyl-X-Verbindung mit einem Alkyl-, Alkenyl-, Cycloalkyl- oder Cycloalkenylhalogenid**
Method for producing organic compounds by means of a transition metal-catalysed cross-coupling reaction of an aryl-X, heteroaryl-X, cycloalkenyl-X or alkenyl-X compound with an alkyl, alkenyl, cycloalkyl or cycloalkenyl halogenide
Procédé de fabrication de liaisons organiques par une réaction de couplage croisé catalysé par du métal de transition d'une liaison d'aryle X, d'hétéroaryle X, de cycloalkényle X ou d'alkényle X et d'une halogénure d'alkyle, d'alkényle, de cycloalkyle ou de cycloalkényle

(30) Priorität: 31.03.2008 DE 102008016702; 17.12.2008 DE 102008062690
(43) Veröffentlichungstag der Anmeldung: 07.10.2009
(73) Patentinhaber: Saltigo GmbH, 40764 Langenfeld (DE)
(72) Erfinder: Sundermeier, Mark, 40225, Düsseldorf (DE); Gotta, Matthias, 51061, Köln (DE); Jacobi von Wangelin, Axel, 50931, Köln (DE); Czaplik, Waldemar Maximilian, 51399, Burscheid (DE)
(74) Vertreter: Pettrich, Klaus-Günter

(56) Entgegenhaltungen:
- EP-A- 1 724 248
- US-A1- 2003 220 498
- CAHIEZ G ET AL: "Cobalt-Catalyzed Alkenylation of Organomagnesium Reagents" TETRAHEDRON LETTERS, ELSEVIER, AMSTERDAM, Bd. 39, Nr. 34, 20. August 1998 (1998-08-20), Seiten 6159-6162, XP004128904 ISSN: 0040-4039
- OHMIYA ET AL: "Cobalt(diamine)-catalysed cross-coupling reaction of alkyl halides with arylmagnesium reagents: stereoselective constructions of arylated asymmetric carbons and application to total synthesis of AH13205" JOURNAL OF THE AMERICAN CHEMICAL SOCIETY., Bd. 128, Nr. 6, 2006, Seiten 1886-1889, XP002542135 XXXX
- VON WANGELIN ET AL: "Domino Iron catalysis: direct aryl-alkyl cross-coupling" ANGEWANDTE CHEMIE. INTERNATIONAL EDITION., Bd. 48, 2009, Seiten 607-610, XP002542136 DEVCH VERLAG, WEINHEIM.

## Beschreibung

Gegenstand der Erfindung ist ein Verfahren zur Herstellung organischer Verbindungen, insbesondere funktionalisierter Aryl-, Heteroaryl-, Cycloalkenyl- oder Alkenylverbindungen, durch eine Übergangsmetall-katalysierte Kreuzkupplungsreaktion einer gegebenenfalls substituierten Aryl-X-, Heteroaryl-X-, Cycloalkenyl-X- oder Alkenyl-X-Verbindung mit einem Alkyl-, Alkenyl-, Cycloalkyl- oder Cycloalkenylhalogenid, wobei X für eine Halogenid-, Diazonium-, Tosylat- (*p-*Toluolsulfonat), Mesylat- (Methansulfonat) oder Triflatabgangsgruppe (Trifluormethansulfonat) steht.

Übergangsmetall-katalysierte Kreuzkupplungen gehören zu den wichtigsten Synthesewerkzeugen in der modernen organischen Chemie. Hierbei finden insbesondere Palladium- und Nickelkatalysatoren auch innerhalb der industriellen Synthese breite Anwendung. So werden zum Beispiel Palladium katalysierte Suzuki-Miyaura Kreuzkupplungen zur Synthese von verschiedenen, pharmakologisch aktiven Substanzen verwendet. Die Synthese von funktionalisierten Styrolen, wie zum Beispiel 4-Chlorstyrol stellen industriell etablierte Kreuzkupplungen auf Basis von Nickelkatalysatoren dar.

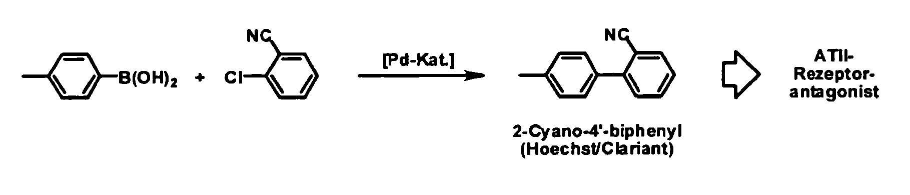

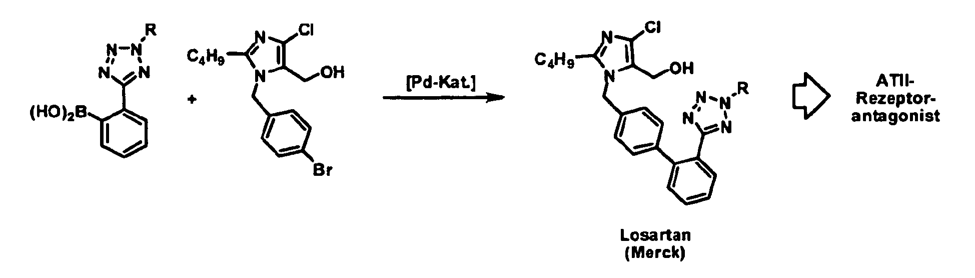

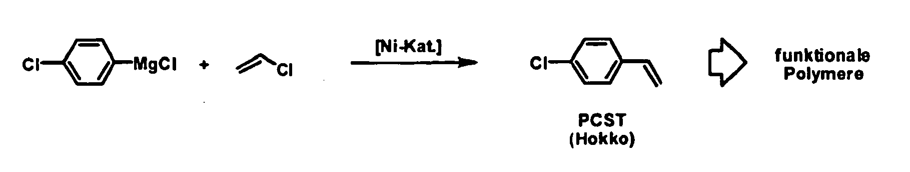

Aus ökonomischen (hohe Weltmarktpreise für Palladium) und aus toxikologischen Gesichtspunkten (hohe Toxizität von Nickel und Palladiumverbindungen) ergeben sich bei der Verwendung der beiden Metalle als Katalysatoren in der industriellen Anwendung deutliche Nachteile. Des Weiteren werden voraussichtlich die Weltmarktspreise für Metalle wie Palladium oder Nickel weiter stark ansteigen und eine industrielle Anwendung in Synthesen immer weiter einschränken. Es wird daher immer stärker nach anderen, katalytisch aktiven Systemen gesucht, welche auf günstige, leicht zugängliche und ungiftige Metalle zurückgreifen.

Neben Nickel- und Palladiumverbindungen weisen auch Eisen- und Kobaltverbindungen unter bestimmten Reaktionsbedingungen Aktivität in Kreuzkupplungsreaktionen auf.

Eisen ist aus ökonomischen- und toxikologischen Gründen den Nickel und Palladiumverbindungen weit überlegen. Eisen wird als das zehnthäufigste Element des Universums geschätzt, allein 5 % der Erdkruste bestehen aus Eisen. Augrund seiner leichten Abbaubarkeit und der Gewinnung aus den Erzen sind viele verschiedene Eisenverbindungen in großen Mengen preisgünstig zu erhalten. Ebenso sind unterschiedliche Kobaltsalze günstig erhältlich und daher z. B. dem Palladium als Katalysatorsystem vorzuziehen.

*Kochi et al.* zeigten bereits Anfang der 70er Jahre das Eisensalze die Kreuzkupplung von Vinylhalogeniden mit Alkylgrignardverbindungen katalysieren können (Kochi et al., J. Am. Chem. Soc. (1971), 1487).

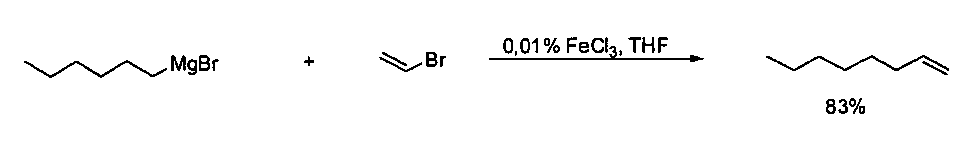

Aufgrund des schmalen Substratspektrums und daher einer eher geringen Anwendungsbreite der Methode fand sie nur wenig Resonanz. Über 30 Jahre später haben unter anderen Arbeiten von *Knochel, Fürstner, Cahiez* und *Nakamura* die Eisen-katalysierte Kreuzkupplung durch Einsatz von stickstoffhaltigen Additiven wie *N*-Methyl-2-pyrrolidon (NMP) oder *N,N,N',N'-*Tetramethylethylendiamin (TMEDA) optimiert und so in den Blickpunkt aktueller Forschung gebracht.

Besonders die milden Reaktionsbedingungen (-20 °C bis 35 °C), die kurzen Reaktionszeiten (in der Regel weniger als 60 Minuten) und das breite Substratspektrum machen Eisen-katalysierte Kreuzkupplung als Synthesewerkzeug äußerst interessant.

*Fürstner et al.* synthetisierten mehrere Naturstoffe (unter anderem FTY720 and *(R)-(+)-*Muscopyridin) unter Verwendung von Eisen-katalysierten Kreuzkupplungen (Fürstner, Chem. Lett. (2005), 624). 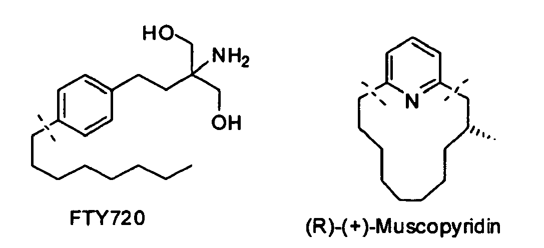 FTY720 and (R)-Muscopyridin.

Für die katalytische Aktivität ist nach *Fürstner et al.* ein kovalenter Eisen-Magnesium-Cluster der formellen Zusammensetzung Fe(MgX)₂ verantwortlich. In diesem Cluster trägt das Eisenatom die formelle Oxidationszahl minus zwei (Superferrat-Cluster).

*Fürstner et al.* entwickelten weiterhin eine Methode zur Kreuzkupplung von funktionalisierten Arylchloriden und -triflaten mit Alkylmagnesiumverbindungen unter Katalyse von 5 mol% Eisen(III)-acetylacetonat und eines THF/NMP Lösungsmittelgemisches (Fürstner, Angew. Chem.(2002), 632). 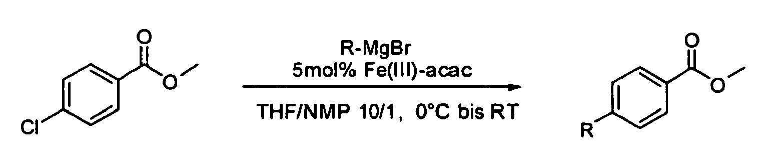 R = Hexyl (95), Isopropyl (59), 6-Hexenyl (91), Phenyl (28)
Ausbeuten in Klammern (GC, %)

Diese Methode erlaubt die Verwendung von Estern, Aminen und chlorierten Heteroaromaten und ermöglicht so eine breitere Anwendung in der Synthese von Naturstoffen.

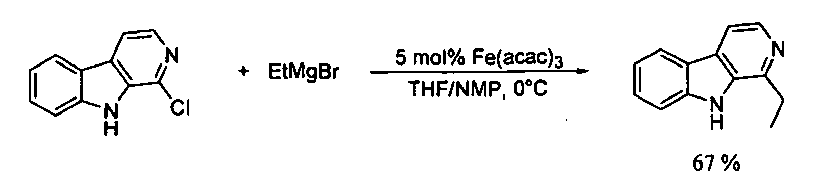

*Nakamura et al.* entwickelten 2004 eine Kreuzkupplung von Alkylhalogeniden mit Arylmagnesium-Verbindungen unter Verwendung von 5 mol% Eisen(III)-chlorid und TMEDA (*N,N,N',N'*-Tetramethylethylendiamin) als Additiv (Nakamura, J. Am. Chem. Soc. (2004), 3686).

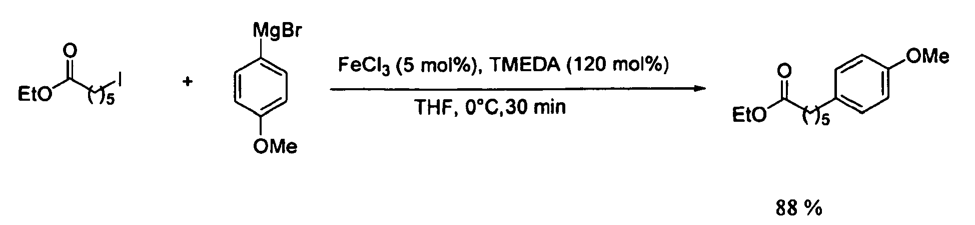

*Knochel et al.* veröffentlichten 2001 eine Kreuzkupplung von hoch funktionalisierten Aryl-Magnesiumverbindungen, die über einen Transmetallierungsschritt zuvor gebildet werden. Diese können in einer weiteren Transmetallierung in stabilere Kupferorganyle umgesetzt werden oder bei niedrigerer Temperatur direkt in einer Kreuzkupplung mit Vinylbromiden oder -iodiden verwendet werden (Knochel, Synlett (2001), 1901). 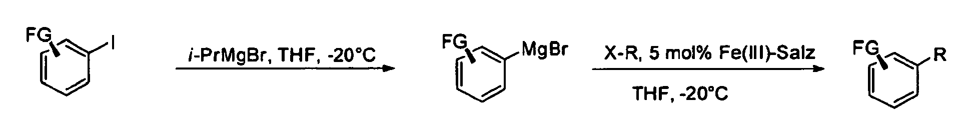 X=Br,l
R = Vinyl
FG = CO₂Et, CN, OTIPS, ONF

*Cahiez et al.* entwickelten 2007 eine effiziente Eisen-katalysierte Kreuzkupplung unter Verwendung von katalytischen Mengen an TMEDA (*N,N,N',N'*-Tetramethylethylendiamin) (10 mol%) und Hexamethylentetramin (5 mol%). Diese Methode ist vor allem geeignet für die Kupplung von Arylgrignardverbindungen mit sekundären und primären Halogeniden. Sekundäre Halogenide liefern hierbei in der Regel bessere Ausbeuten an Kupplungsprodukt (Cahiez, Angew. Chem. Int. Ed. (2007), 4364).

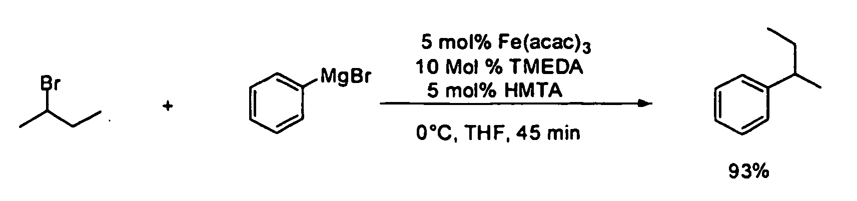

Durch Einsatz eines nicht hygroskopischen (FeCl₃)₂(TMEDA)₃ Komplex konnte die Katalysatorenmenge auf 1.5 mol% gesenkt werden. Der leicht herstellbare und lagerfähige Komplex ermöglicht eine sehr einfache aber effiziente Kreuzkupplung.

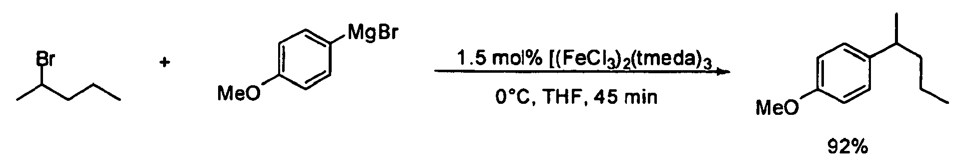

Die ersten effizienten Kobalt-katalysierten Kreuzkupplungen von Alkenylhalogeniden mit Alkylgrignardverbindungen wurden Ende der 1990er Jahre von *Cahiez et al.* veröffentlicht. Als Katalysatorsystem dienten CoCl₂ und Co(acac)₂ in einem NMP/THF-Lösungsmittelgemisch (Cahiez, Tetrahedron Lett. (1998), 6159).

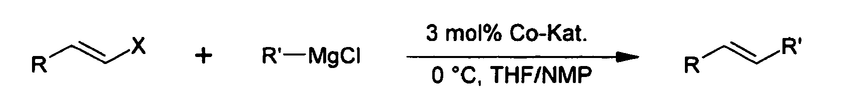

*Knochel et al.* erweiterten diese Kobalt-katalysierte Kreuzkupplungsmethode auf die Umsetzung von Alkenyl- und Arylhalogeniden mit Organokupfer- und Organozinkverbindungen (Knochel, Tetrahedron Lett. (1998), 6163; Angew. Chem. (2005), 3007).

Die erste stereoselektive Variante mit moderaten Selektivitäten in der Kobalt-katalysierten Kreuzkupplung eines Alkylhalogenids mit einer Arylgrignardverbindung wurde 2006 von *Oshima et al.* beschrieben. Es wurde auch der Einfluss eines benachbarten Chiralitätszentrums am Alkylhalogenids auf die Diastereoselektivität der Kreuzkupplung untersucht. Weiterhin wurde gezeigt, dass neben Aminen auch Diphosphine als Liganden in der Kobalt-katalysierten Kreuzkupplung von Alkylhalogeniden mit Arylgrignardverbindungen geeignet sind. (Oshima, J. Am. Chem. Soc. (2006), 1886; Tetrahedron (2006), 2207).

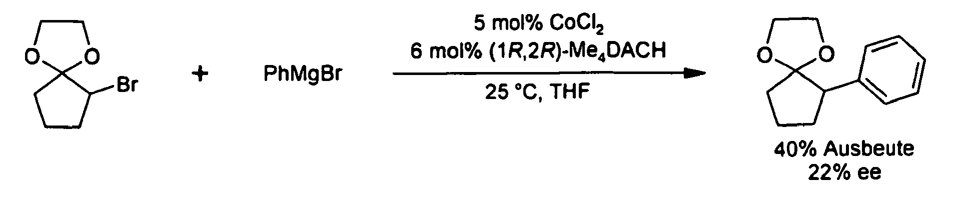

Im Jahr 2007 konnten *Hayashi et al.* zeigen, dass neben Alkyl-, Alkenyl- und Arylgrignardverbindungen auch Alkinylgrignardverbindungen in der Kobalt-katalysierten Kreuzkupplung mit Alkenyltriflaten eingesetzt werden können (Hayashi, Chem. Comm. (2007), 4513).

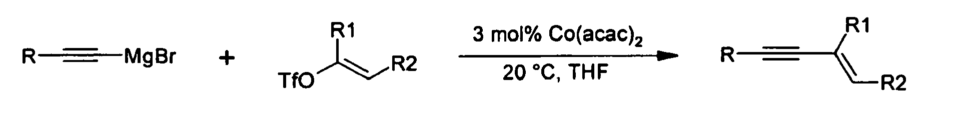

Die zuvor genannten Eisen- und Kobalt-katalysierten Kreuzkupplungen basieren allesamt auf der Kupplung einer Organomagnesiumverbindung (Grignardverbindung) mit einem Alkyl-, Alkenyl- oder Arylhalogenid.

Die Grignardverbindung muss dabei vorher getrennt hergestellt werden und wie zum Beispiel nach *Nakamura et al.* über einen längeren Zeitraum langsam zugetropft werden (Nakamura, J. Am.Soc. (2004), 3686). Gerade im größeren Maßstab ist das Lagern von pyrophoren Grignardverbindungen bedenklich und der Umgang mit Ihnen aufgrund ihrer Empfindlichkeit gegenüber Luftfeuchtigkeit schwierig. Aufgrund dieses Gefahrenpotenzials sind besondere Anforderungen an die Prozesssicherheit bei der industriellen Anwendung erforderlich, was eine Umsetzung im großen Maßstab deutlich erschwert.

Aufgabe der vorliegenden Erfindung war es daher, ein Verfahren zur Herstellung organischer Verbindungen aufzufinden, bei dem keine Organomagnesiumverbindung (Grignardverbindung) separat hergestellt und isoliert werden muss.

Es wurde nun ein Verfahren gefunden, das eine Übergangsmetall-katalysierte Bildung einer Grignardverbindung mit einer direkten Kreuzkupplung mit einem Organohalogenid in einer Eintopfreaktion miteinander vereint. Die Gefahren die mit der Lagerung und einer separaten Herstellung von Organomagnesiumverbindungen verbunden sind, entfallen somit im erfindungsgemäßen Verfahren. Des Weiteren kann durch Ausnutzen nur eines Reaktionsgefäßes die Effizienz und Raumausnutzung beim erfindungsgemäßen Verfahren im Vergleich mit herkömmlichen Verfahren deutlich gesteigert werden.

Gegenstand der Erfindung ist daher ein Verfahren zur Herstellung organischer Verbindungen der allgemeinen Formel (I)

R-R' (I),

worin
- R: für einen gegebenenfalls substituierten aromatischen, heteroaromatischen, cycloalkenylischen oder alkenylischen Rest steht, und
- R': für einen gegebenenfalls substituierten alkylischen, alkenylischen, cycloalkylischen oder cycloalkenylischen Rest steht,
durch Umsetzung einer entsprechenden Verbindung der allgemeinen Formel (II)

R-X (II),

worin
- X: für Chlor, Brom, Iod, Diazonium, Mesylat (Methansulfonat), Tosylat (*p*- Toluolsulfonat) oder Triflat (Trifluormethansulfonat) steht, und
- R: die für Formel (I) angegebene Bedeutung hat,
mit einer entsprechenden Verbindung der allgemeinen Formel (III)

R'-Y (III)

worin
- Y: für Chlor, Brom oder Iod steht, und
- R': die für Formel (I) angegebene Bedeutung hat,
dadurch gekennzeichnet, dass man die Reaktion in Gegenwart
a) stöchiometrischer Mengen elementaren Magnesiums, bezogen auf die Verbindung der allgemeinen Formel (II) und
b) katalytischer Mengen einer Übergangsmetallverbindung, bezogen auf die Verbindung der allgemeinen Formel(II), wobei die Übergangsmetallverbindung eine Eisenverbindung aus der Gruppe Eisen(II)chlorid, Eisen(III)chlorid, Eisen(II)acetylacetonat, Eisen(III)acetylacetonat, Eisen(II)acetat, Eisen(III)acetat, Eisen(II)bromid, Eisen(III)bromid, Eisencarbonyl-Komplex oder Kobalt(II)chlorid oder Kobalt(II)bromid ist,
   sowie gegebenenfalls in Gegenwart
c) eines stickstoff-, sauerstoff- und/oder phosphorhaltigen Additivs in katalytischer oder stöchiometrischer Menge bezogen auf die Verbindung der allgemeinen Formel (II),
   und die Reaktion als Eintopfverfahren durchgerührt wird, bei der die *in situ* als Intermediat entstehende Organomagnesiumverbindung (Grignard-Verbindung) nicht isoliert wird.

Besonders vorteilhaft ist beim erfindungsgemäßen Verfahren, das dieses als Eintopfverfahren durchgeführt wird und die dabei als Intermediat *in situ* gebildete Organomagnesiumverbindung (Grignardverbindung) nicht isoliert wird.

Die Übergangsmetallverbindungen, insbesondere die genannten Eisen- und Kobaltverbindungen katalysieren die Bildung von Organomagnesiumverbindungen aus Halogeniden bei niedrigen Temperaturen. Die so gebildeten Grignardverbindungen werden *in situ* erfindungsgemäß direkt mit einer Aryl-X-Verbindung, Heteroaryl-X-Verbindung oder Alkenyl-X-Verbindung unter Verwendung des gleichen katalytischen Systems in einer Kreuzkupplungsreaktion weiter umgesetzt. Die Gruppe X in Formel (II) ist dabei eine Abgangsgruppe, welche ein Halogenid, Diazonium, Mesylat, Tosylat oder Triflat sein kann. Dabei bleibt die Konzentration an aktiver Grignardverbindung aufgrund der schnellen Folgereaktion nur sehr gering. Experimente haben bestätigt, dass im erfindungsgemäßen Verfahren während der Reaktion eine Konzentration von 5% aktiver Grignardverbindung nicht überschritten wird. Mit hoher Selektivität wird hierbei bevorzugt der Alkylgrignard gebildet.

Die Handhabung und langsame Dosierung einer isolierten, pyrophoren Grignardverbindung ist durch die langsame und gleichmäßige Bildung des aktiven Grignards *in situ* nicht mehr notwendig. Daher wird durch Anwendung des erfindungsgemäßen Verfahrens das Gefahrenpotenzial bei einem großtechnischen, industriellen Prozess deutlich gesenkt und die Prozesssicherheit damit erheblich verbessert.

Bei dem Rest R in Formel (I) bzw. Formel (II) handelt es sich um einen gegebenenfalls substituierten alkenylischen, cycloalkenylischen, aromatischen oder heteroaromatischen Rest, wobei der heteroaromatische Rest ein fünf-, sechs- oder siebengliedriger Ring mit einem oder mehreren Stickstoff-, Sauerstoff- und/oder Schwefelatomen im Ring bedeutet. An dem cycloalkenylischen, aromatischen und dem heteroaromatischen Rest können gegebenenfalls weitere aromatische, heteroaromatische und/oder cycloaliphatische Reste ankondensiert sein.

Beispiele für aromatische Reste R sind Phenyl-, Naphthyl-, Tolyl-, Anisol-, Kresol-, Anilin oder Benzoesäurereste. Ein Beispiel für einen heteroaromatischen Rest sind Pyridinreste. Beispiele für alkenylische Reste R sind 2-Methylpropenyl- oder 2-Phenylethenylreste. Ein Beispiel für einen cycloalkenylische Rest ist der 1-Cyclohexenylrest.

Der alkenylische, cycloalkenylische, aromatische oder heteroaromatische Rest R kann bis zu acht Substituenten tragen, welche unabhängig voneinander (C)-C₁₂)-Alkyl, (C₁-C₁₂)-Cycloalkyl, (C₁-C₁₂)-Alkenyl, (C₁-C₁₂)-Cycloalkenyl, (C₁-C₁₂)-Alkinyl, (C₁-C₁₂)-Aryl, O-[(C₁-C₁₂)-Alkyl], O-[(C₁-C₁₂)-Aryl], O-Si[(C₁-C₁₂)-Alkyl]ₙ[C₁-C₁₂)-Aryl]₃₋ₙ, OC(O)-[(C₁-C₁₂)-Alkyl], OC(O)-[(C₁-C₁₂)-Aryl], NH₂, NH[(C₁-C₁₂)-Alkyl], N[(C₁-C₁₂)-Alkyl]₂, NH[(C₁-C₁₂)-Aryl], N[(C)-C₁₂)-Aryl]₂, NHC(O)-[(C₁-C₁₂)-Alkyl], N[(C₁-C₁₂)-Alkyl]C(O)-[(C₁-C₁₂)-Alkyl], NHC(O)-[(C₁-C₁₂)-Aryl], N[(C₁-C₁₂)-Alkyl]C(O)-[(C)-C₁₂)-Aryl], NO₂, NO, S-[(C₁-C₁₂)-Aryl], S-[(C₁C₁₂)-Alkyl], Fluor, Chlor, Brom, CF₃, CN, COOM, COO-[(C₁-C₁₂)-Alkyll, COO-[(C₁-C₁₂)-Aryl], C(O)NH-[(C₁-C₁₂)-Alkyl], C(O)NH-[(C₁-C₁₂)-Aryl], C(O)N-[(C₁-C₁₂)-Alkyl]₂, C(O)N-[(C₁-C₁₂)-Aryl]₂, CHO, SO₂-[(C₁-C₁₂)-Alkyl], SO-[(C₁C₁₂)-Alkyl], SO₂-[(C₁-C₁₂)-Aryl], SO-[(C₁-C₁₂)-Aryl], OSO₂-[(C₁-C₁₂)-Alkyl], OSO₂-[(C₁-C₁₂)-Aryl], PO-[(C₁-C₁₂)-Alkyl]₂, PO-[(C₁-C₁₂)-Aryl]₂, SO₃M, SO₃-[(C₁-C₁₂)-Alkyl], SO₃-[(C₁-C₁₂)-Aryl] oder Si[(C₁-C₁₂)-Alkyl]ₙ[C₁-C₁₂]-Aryl]₃₋ₙ bedeuten können, wobei M für ein Alkali- oder Erdalkaliatom und n für eine natürliche Zahl im Bereich von 0 bis 3 steht.

Der alkylische, alkenylische, cycloalkylische oder cycloalkenylische Rest R' in Formel (I) bzw. Formel (II) kann gegebenenfalls ein oder mehrere Substituenten tragen, welche unabhängig voneinander (C₁-C₁₂)-Alky), (C₁-C₁₂)-Cycloalkyl, (C₁-C₁₂)-Alkenyl, (C₁-C₁₂)-Cycloalkenyl, (C₁-C₁₂)-Alkinyl, (C₁-C₁₂)-Aryl, O-[(C₁-C₁₂)-Alkyl], O-[(C₁-C₁₂)-Aryl], O-Si[(C₁-C₁₂)-Alkyl]ₙ[C₁-C₁₂)-Aryl]₃₋ₙ, OC(O)-[(C₁-C₁₂)-Alkyl], OC(O)-[(C₁-C₁₂)-Aryl], NH₂, NH[(C₁-C₁₂)-Alkyl], N[(C₁-C₁₂)-Alkyl]₂, NH[(C₁-C₁₂)-Aryl], N[(C₁-C₁₂)-Aryl]₂, NHC(O)-[(C₁-C₁₂)-Alkyl], N[(C₁-C₁₂)-Alkyl]C(O)-[(C₁-C₁₂)-Alkyl], NHC(O)-[(C₁-C₁₂)-Aryl], N[(C₁-C₁₂)-Alkyl]C(O)-[(C₁-C)₂)-Aryl], NO₂, NO, S-[(C₁-C₁₂)-Aryl], S-[(C₁-C₁₂)-Alkyl], Fluor, Chlor, Brom, CF₃, CN, COOM, COO-[(C₁-C₁₂)-Alkyl], COO-[(C₁-C₁₂)-Aryl], C(O)NH-[(C₁-C₁₂)-Alkyl], C(O)NH-[(C₁-C₁₂)-Aryl], C(O)N-[(C₁-C₁₂)-Alkyl]₂, C(O)N-[(C)-C₁₂)-Aryl]₂, CHO, SO₂-[(C₁-C₁₂)-Alkyl], SO-[(C₁-C₁₂)-Alkyl], SO₂(C₁-C₁₂)-Aryl], SO-[(C₁-C₁₂)-Aryl], OSO₂-[(C₁-C₁₂)-Alkyl], OSO₂-[(C₁-C₁₂)-Aryl], PO-[(C₁-C₁₂)-Alkyl]₂, PO-[(C₁-C₁₂)-Aryl]₂, SO₃M, SO₃-[(C₁-C₁₂)-Alkyl], SO₃-[(C₁-C₁₂)-Aryl) oder Si[(C₁-C₁₂)-Alkyl]ₙ[C₁-C₁₂)-Aryl]₃₋ₙ bedeuten können, wobei M für ein Alkali- oder Erdalkaliatom und n für eine natürliche Zahl im Bereich von 0 bis 3 steht.

Beispiele für alkylische Reste R' sind lineare und verzweigte, gegebenenfalls substituierte C₃-C₁₂-Alkyle, für cycloalkylische Reste Cyclohexyl, für alkenylische Reste lineare und verzweigte, gegebenenfalls substituierte C₃-C₁₂-Olefine und für cycloalkenylische Reste 1-Cyclohexenyl.

Es werden Eisenverbindungen aus der Gruppe Eisen(II)chlorid, Eisen(III)chlorid, Eisen(II)acetylacetonat, Eisen(III)acetylacetonat, Eisen(II)acetat, Eisen(III)acetat, Eisen(II)bromid, Eisen(III)bromid, Eisencarbonyl-Komplexe oder Kobalt(II)chlorid oder Kobalt(II)bromid eingesetzt.

Die eingesetzte Katalysatormenge beträgt dabei bevorzugt 0,01 bis 50 mol%, besonders bevorzugt 0,1 bis 10 mol%, bezogen auf die Verbindung der allgemeinen Formel (II).

Gewünschtenfalls können im erfindungsgemäßen Verfahren Additive zugesetzt werden (Punkt c).

Bei den gegebenenfalls zugesetzten stickstoff-, sauerstoff- und/oder phosphorhaltigen Additiven mit einem oder mehreren Stickstoff-, Sauerstoff- und/oder Phosphoratomen handelt es sich bevorzugt um gegebenenfalls substituierte Alkylamine, *N*-haltige Heterocyclen, Alkylamide, cyclische Alkylamide, Cycloalkylamine, Cycloalkyldiamine, Alkylimine, Cycloalkylimine, Anilin, Anilin-Derivate, stickstoffhaltige Heteroaromaten, Dialkylether, Alkylarylether, Diarylether, cyclische Ether, Oligoether, Polyether, Triarylphosphane, Trialkylphosphane, Aryldialkylphosphane, Alkyldiarylphosphane und verbrückte Bisphosphane.

Besonders bevorzugt werden TEA (Triethylamin), Ethyl-diisopropylamin, TMEDA (*N,N,N',N'-*Tetramethylethylendiamin), DABCO (1,4-Diazabicyclo[2.2.2]octan), (-)-Spartein, *N,N,N',N'-*Tetramethyldiaminomethan, DACH (1,2-Diaminocyclohexan), Me₄-DACH (*N,N,N',N'-*Tetramethyl-1,2-diaminocyclohexan), NMP (*N*-Methyl-2-pyrrolidon), *N,N-*Dimethylanilin, Pyridin, Phenanthrolin, PEG (Polyethylenglycol), DME (1,2-Dimethoxyethan) Binaphthyldimethylether, 18-Krone-6, Triphenylphosphan, Tri-n-butylphosphan, Tri-tert-butylphosphan, dppf (1,1'-Bis(diphenylphosphino)ferrocen), dppe (1,2-Bis(diphenylphosphino)ethan), dppp (1,3-Bis(diphenylphosphino)propan), dppb (1,4-Bis(diphenylphosphino)butan) oder dpppe (1,5-Bis(diphenylphosphino)pentan als Additiv eingesetzt.

Der Einsatz von chiralen stickstoff-, sauerstoff- und/oder phosphorhaltigen Additiven mit einem oder mehreren Stickstoff-, Sauerstoff- und/oder Phosphoratomen beim erfindungsgemäßen Verfahren führt bei geeigneter Wahl der Substrate zur selektiven Bildung eines Stereozentrums der Formel (IVa) oder Formel (IVb) in stereoisomerenangereicherten Form, worin R die für Formel (I) angegebene Bedeutung hat und R1, R2 und R3 unabhängig voneinander die folgende Bedeutung haben können: Wasserstoff, (C₁-C₁₂)-Alkyl, (C₁-C₁₂)-Cycloalkyl, (C₁-C₁₂)-Alkenyl, (C₁-C₁₂)-Cycloalkenyl, (C₁-C₁₂)-Alkinyl, (C₁-C₁₂)-Aryl, O-[(C₁-C₁₂)-Alkyl], O-[(C₁-C₁₂)-Aryl], O-Si[(C₁-C₁₂)-Alkyl]ₙ[C₁C₁₂)-Aryl]₃₋ₙ OC(O)-[(C₁-C₁₂)-Alkyl], OC(O)-[(C₁-C₁₂)-Aryl), NH₂, NH[(C₁-C₁₂)-Alkyl], N[(C₁-C₁₂)-Alkylh, NH[(C₁-C₁₂)-Aryl], N[(C₁-C₁₂)-Aryl]₂, NHC(O)-[(C₁-C₁₂)-Alkyl], N[(C₁-C₁₂)-Alkyl]C(O)-[(C₁-C₁₂)-Alkyl], NHC(O)-[(C₁-C₁₂)-Aryl], N[(C₁-C₁₂)-Alkyl]C(O)-[(C₁-C₁₂)-Aryl], NO₂, NO, S-[(C₁-C₁₂)-Aryl], S-[(C₁-C₁₂)-Alkyl], Fluor, Chlor, Brom, CF₃, CN, COOM, COO-[(C₁-C₁₂)-Alkyl], COO-[(C₁-C₁₂)-Aryl], C(O)NH-[(C₁-C₁₂)-Alkyl], C(O)NH-[(C₁-C₁₂)-Aryl], C(O)N-[(C₁-C₁₂)-Alkyl]₂, C(O)N-[(C₁-C₁₂)-Aryl]₂, CHO, SO₂-1(C₁-C₁₂)-Alkyl], SO-[(C₁-C₁₂)-Alkyl], SO₂-[(C₁-C₁₂)-Aryl], SO-[(C₁-C₁₂)-Aryl], OSO₂(C₁-C₁₂)-Alkyl], OSO₂-[(C₁-C₁₂)-Aryl], PO-[(C₁-C₁₂)-Alkyl]₂, PO-[(C₁-C₁₂)-Aryl]₂, SO₃M, SO₃-[(C₁-C₁₂)-Alkyl], SO₃-[(C₁-C₁₂)-Aryl] oder Si[(C₁-C₁₂)-Alkyl]ₙ[C₁-C₁₂)-Aryl]₃₋ₙ bedeuten können, wobei M für ein Alkali- oder Erdalkaliatom und n für eine natürliche Zahl im Bereich von 0 bis 3 steht. Es muss jedoch R ≠ R1 ≠ R2 ≠ R3 erfüllt sein. R, R1, R2 und/oder R3 können unabhängig voneinander über die zuvor genannte Substituenten miteinander verbrückt sein. Das Kohlenstoffatom zusammen mit den Resten R1, R2 und R3 entspricht R' mit der für Formel (I) angegebenen Bedeutung.

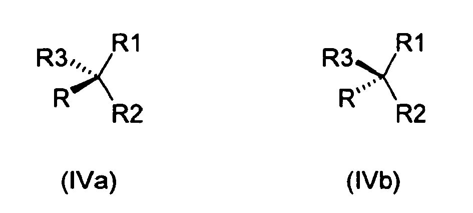

Die Bildung des einen oder anderen Stereoisomers in stereoisomerenangereicherter Form wird im erfindungsgemäßen Verfahren gezielt durch die Wahl des chiralen Liganden/Additiv gesteuert.

Als chirale Liganden werden stickstoff-, sauerstoff- und/oder phosphorhaltigen Additive mit einem oder mehreren Stickstoff-, Sauerstoff- und/oder Phosphoratomen, bevorzugt gegebenenfalls substituierte Alkylamine, *N*-haltige Heterocyclen, Alkylamide, cyclische Alkylamide, Cycloalkylamine, Cycloalkyldiamine, Alkylimine, Cycloalkylimine, Anilin, Anilin-Derivate, stickstoffhaltige Heteroaromaten, Dialkylether, Alkylarylether, Diarylether, cyclische Ether, Oligoether, Polyether, Triarylphosphane, Trialkylphosphane, Aryldialkylphosphane, Alkyldiarylphosphane und verbrückte Bisphosphane mit einem oder mehreren Chiralitätszentren eingesetzt.

Im erfindungsgemäßen Verfahren wird das stickstoff-, sauerstoff- und/oder phosphorhaltige Additiv bevorzugt in einer Menge von 0 bis 200 mol%, besonders bevorzugt 1 bis 150 mol%, bezogen auf die Verbindungen (II) eingesetzt.

Das erfindungsgemäße Verfahren wird üblicherweise in aprotisch polaren Lösungsmitteln durchgeführt. Besonders bevorzugt wird dabei Tetrahydrofuran (THF), 2-Methyltetrahydrofuran (2-Methyl-THF), 1,4-Dioxan, Dimethylformamid (DMF), Dimethylacetamid (DMAc), Methyl-tert-butylether (MTBE), Diethylether, 1,2-Dimethoxyethan (DME), Diisopropylether (DIPE), Dimethylcarbonat oder *N*-Methyl-2-pyrrolidon (NMP) als Lösungsmittel verwendet.

Die Reaktionstemperatur beim erfindungsgemäßen Verfahren liegt üblicherweise zwischen -80 °C und +100 °C.

Nach dem erfindungsgemäßen Verfahren gelingt die Umsetzung einer Vielzahl von substituierten und unsubstituierten Aryl-X-, Heteroaryl-X-, Cycloalkenyl-X- und Alkenyl-X-Verbindungen, wobei X für einen Abgangsgruppe wie Halogenid, Diazonium, Mesylate, Tosylat oder Triflat steht, mit substituierten und unsubstituierten Alkyl-, Alkenyl-, Cycloalkyl- oder Cycloalkenylhalogeniden zu den entsprechenden funktionalisierten Aromaten, Heteroaromaten und Olefinen.

Die nach dem erfindungsgemäßen Verfahren hergestellten Verbindungen lassen sich nach herkömmlichen Methoden gut isolieren und aufreinigen.

Das erfindungsgemäße Verfahren eignet sich sehr gut, um gezielt und selektiv Chiralitätszentren mittels Kreuzkupplung zu erzeugen.

### Beispiele

### Beispiel 1 bis 15

Bei einer Temperatur von 0 °C werden in einem 10 ml Reaktionskolben 29 mg (1,2 mmol) Magnesiumspäne vorgelegt und mit Argon gespült. Es wird eine Lösung aus 8,1 mg FeCl₃ (0,005 mmol; 5 mol%) und 4 ml THF (wasserfrei) zugegeben und im Anschluss 181 µl TMEDA (1,2 mmol) zudosiert. Die Mischung wird bei einer Temperatur von 0 °C 30 min gerührt und dann die Verbindung R-X (1 mmol) und die Verbindung R'-Y (1,2 mmol) dazugegeben. Die Reaktionsmischung wird 3 Stunden bei Raumtemperatur gerührt und anschließend durch Zugabe von 3 ml einer gesättigten wässrigen Ammoniumchloridlösung und 1 ml 10 %ige wässrige HCl gestoppt. Es wird mit 3x 5 ml Ethylacetat extrahiert, die vereinigten organischen Phasen über Natriumsulfat getrocknet und das Lösungsmittel im Vakuum abdestilliert. Das Rohprodukt wird säulenchromatographisch gereinigt (Kieselgel, Cyclohexan, Ethylacetat).

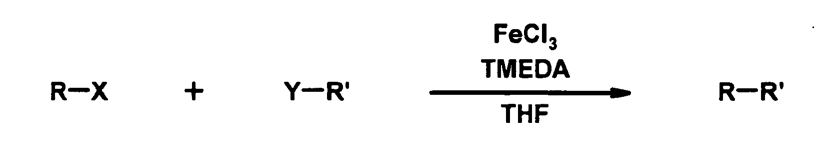

| Beispiel | R-X | R'-Y | R-R' | Ausbeute |
|---|---|---|---|---|
| 1 | 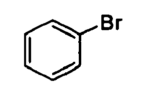 | 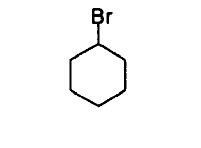 | 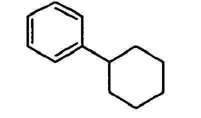 | 77 % |
| 2 | 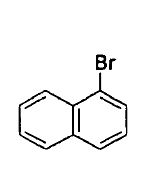 | 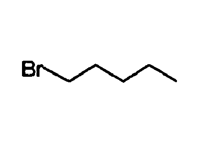 | 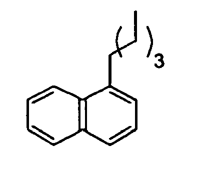 | 71 % |
| 3 | 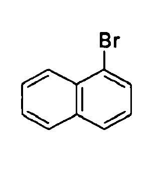 | 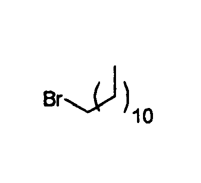 | 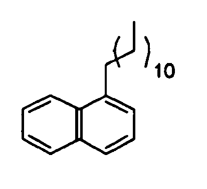 | 75 % |
| 4 | 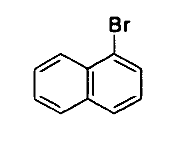 | 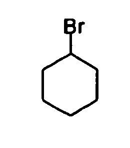 | 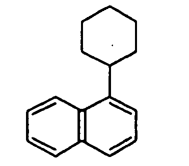 | 67 % |
| 5 | 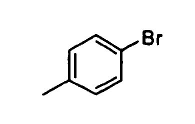 | 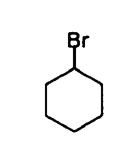 | 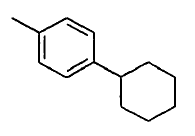 | 77% |
| 6 | 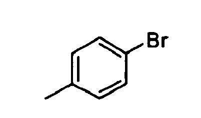 | 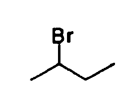 | 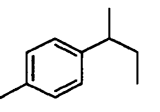 | 62% |
| 7 | 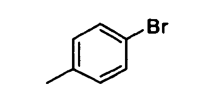 | 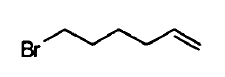 | 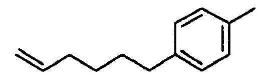 | 55 % |
| 8 | 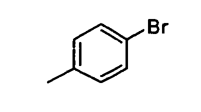 | 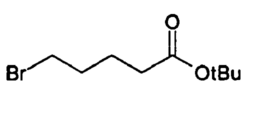 | 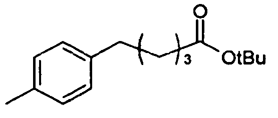 | 40% |
| 9 | 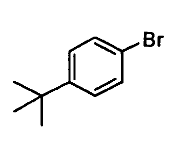 | 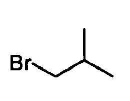 | 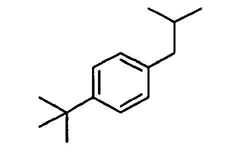 | 67 % |
| 10 | 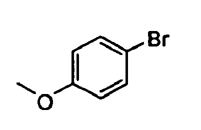 | 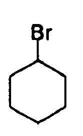 | 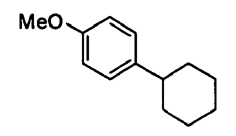 | 65 % |
| 11 | 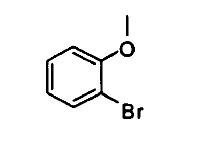 | 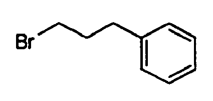 | 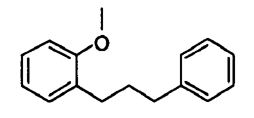 | 74 % |
| 12 | 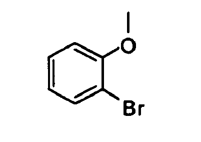 | 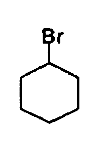 | 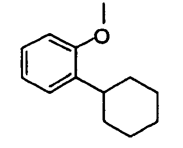 | 69 % |
| 13 | 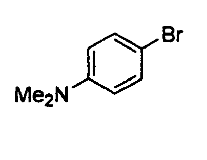 | 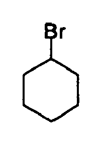 | 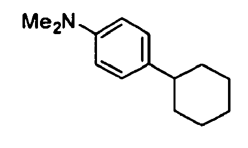 | 75 % |
| 14 | 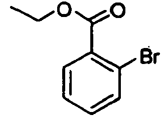 | 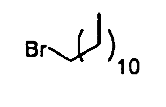 | 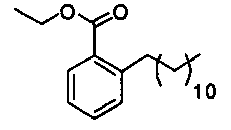 | 40% |
| 15 | 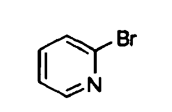 | 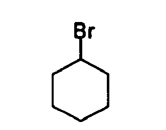 | 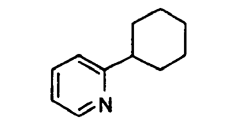 | 55 % |

### Beispiel 16 bis 20

Analog zur Versuchsvorschrift aus Beispiel 1-15 können auch Aryl- und Alkylchloride sowie Aryltriflate erfolgreich eingesetzt werde. Im Fall von Phenyltriflat (Phenyltrifluormethansulfonat) und von Chlorbenzol wurde die Reaktion bei 20 °C durchgeführt.

| Beispiel | R-X | R'-Y | R-R' | Ausbeute |
|---|---|---|---|---|
| 16 | 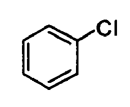 | 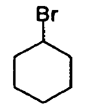 | 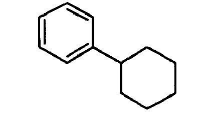 | 20% |
| 17 | 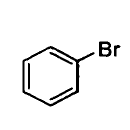 | 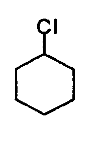 | 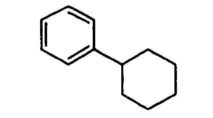 | 25% |
| 18 | 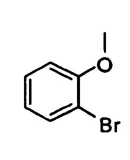 | 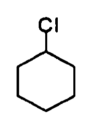 | 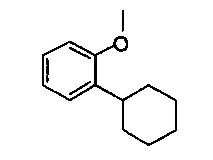 | 39% |
| 19 | 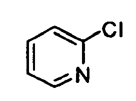 | 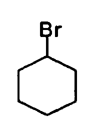 | 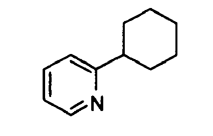 | 30% |
| 20 | 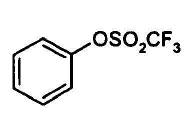 | 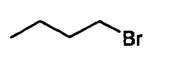 | 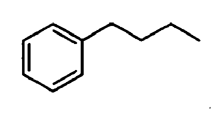 | 80% |

Analog zur Versuchsvorschrift aus Beispiel 1-15gelingt auch die Umsetzung von Alkenylbromiden mit Alkylbromiden.

| Beispiel | R-X | R'-Y | R-R' | Ausbeute |
|---|---|---|---|---|
| 21 | 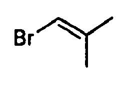 | 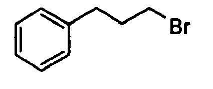 | 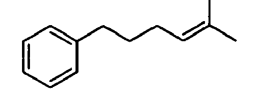 | 48% |

### Beispiel 22

Analog zur Versuchsvorschrift aus Beispiel 1-15, jedoch bei Verwendung von 10 mol% TMEDA anstelle von 120 mol%, gelingt die Umsetzung von 4-Bromtoluol mit 1-Bromdodecan.

| Beispiel | R-X | R'-Y | R-R' | Ausbeute |
|---|---|---|---|---|
| 22 | 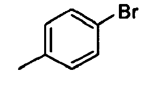 | 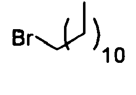 | 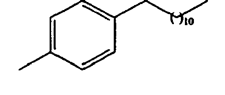 | 53% |

### Beispiel 23 bis 31

Analog zur Versuchsvorschrift aus Beispiel 1-15, wurde 4-Bromanisol mit 1-Bromdodecan umgesetzt. Anstelle von TMEDA wurden elektronisch und sterisch unterschiedliche stickstoffhaltige Additive verwendet.

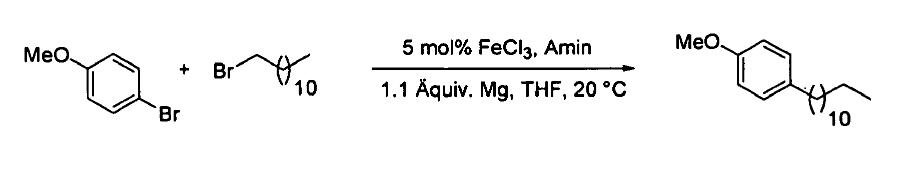

| Beispiel | Additiv | Ausbeute |
|---|---|---|
| 23 | 1,10-Phenanthrolin | 63 % |
| 24 | Pyridin | 72 % |
| 25 | N,N-Dimethylanilin | 49 % |
| 26 | N-Methylpyrrolidon | 39 % |
| 27 | Tetramethyldiaminomethan | 72 % |
| 28 | Ethyl-diisopropylamin | 44 % |
| 29 | TMEDA | 77 % |
| 30 | (-)-Spartein | 36 % |
| 31 | DABCO | 75 % |

### Beispiel 32 bis 35

Analog zu Beispiel 23-31 sind auch sauerstoff- und phosphorhaltige Verbindungen wie Ether und Phosphane als Additive in der Kreuzkupplung von 4-Bromanisol und 1-Bromdodecan geeignet. Wie Beispiel 32 zeigt, gelingt die Reaktion auch in Abwesenheit jeglicher Additive.

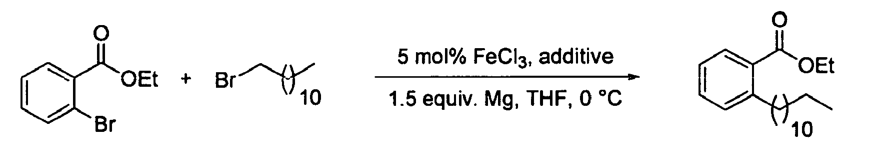

| Beispiel | Additiv | mol% | Ausbeute |
|---|---|---|---|
| 32 | - | - | 30 % |
| 33 | 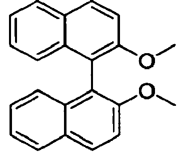 | 20 | 73 % |
| 34 | 18-Krone-6 | 20 | 55 % |
| 35 | PPh₃ | 20 | 51 |

### Beispiel 36 bis 44

Bei Raumtemperatur werden in einem 10 ml Reaktionskolben 29 mg (1,2 mmol) Magnesiumspäne vorgelegt und mit Argon gespült. Es wird eine Lösung aus 6,5 mg CoCl2 (0,05 mmol; 5 mol%) und 4 ml THF (wasserfrei) zugegeben und im Anschluss 19 µl *N,N,N',N*'-Tetramethyl-1,2-diaminocyclohexan (Me4DACH; 0,11 mmol, 11 mol%) zudosiert. Die blaue Mischung wird unter Rühren auf eine Temperatur von 0 °C gekühlt und dann die Verbindung R-X (1,0 mmol) und die Verbindung R'-Y (1,0 mmol) dazugegeben. Die Reaktionsmischung wird 3 Stunden bei 0 °C gerührt und anschließend durch Zugabe von 3 ml einer gesättigten wässrigen Ammoniumchloridlösung gestoppt. Es wird mit 3x 5 ml Ethylacetat extrahiert, die vereinigten organischen Phasen über Natriumsulfat getrocknet und das Lösungsmittel im Vakuum abdestilliert. Das Rohprodukt wird säulenchromatographisch gereinigt (Kieselgel, Cyclohexan, Ethylacetat).

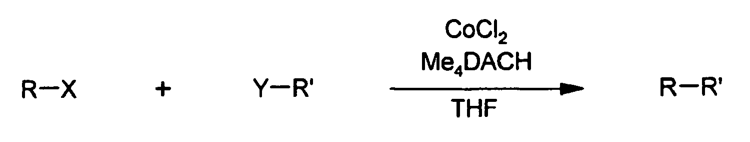

| Beispiel | R-X | R'-Y | R-R' | Ausbeute |
|---|---|---|---|---|
| 36 | 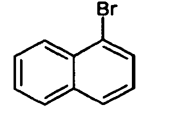 | 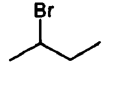 | 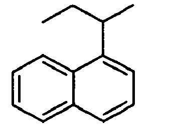 | 64% |
| 37 | 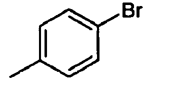 | 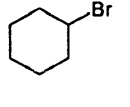 | 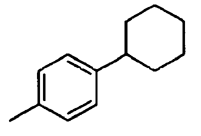 | 67% |
| 38 | 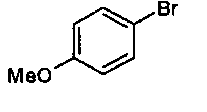 | 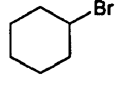 | 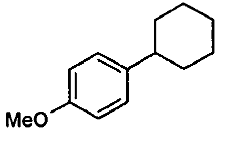 | 52% |
| 39 | 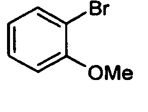 | 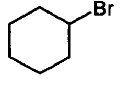 | 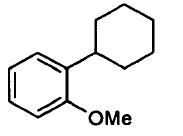 | 60% |
| 40 | 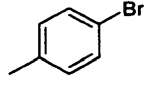 | 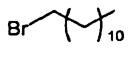 | 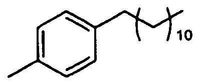 | 66% |
| 41 | 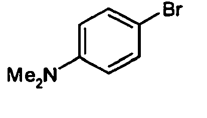 | 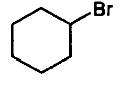 | 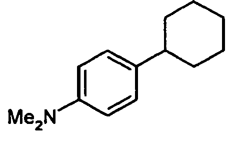 | 42% |
| 42 | 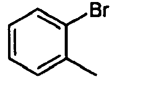 | 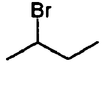 | 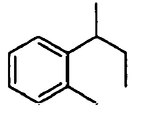 | 52% |
| 43 | 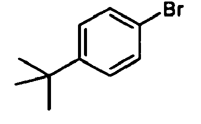 | 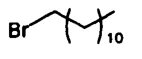 | 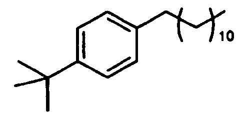 | 53% |
| 44 | 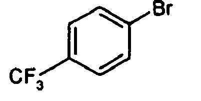 | 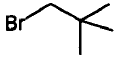 | 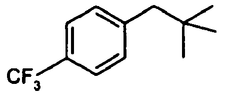 | 46% |

### Beispiel 45 bis 47

Analog zur Versuchsvorschrift aus Beispiel 36-44 können auch Aryl- und Alkylchloride erfolgreich eingesetzt werde.

| Beispiel | R-X | R'-Y | R-R' | Ausbeute |
|---|---|---|---|---|
| 45 | 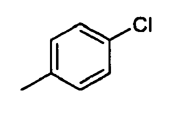 | 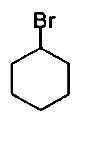 | 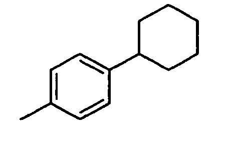 | 8% |
| 46 | 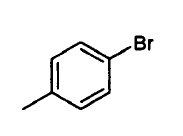 | 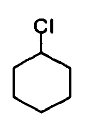 | 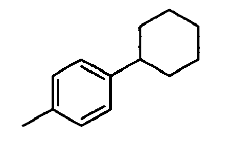 | 42% |
| 47 | 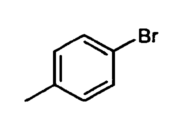 | 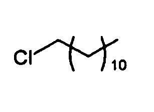 | 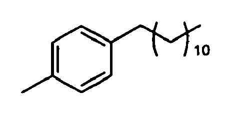 | 7% |

### Beispiel 48

Analog zur Versuchsvorschrift aus Beispiel 36-44 gelingt auch die Umsetzung von Alkenylbromiden mit Alkylbromiden.

| Beispiel | R-X | R'-Y | R-R' | Ausbeute |
|---|---|---|---|---|
| 48 | 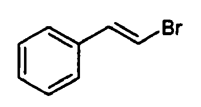 | 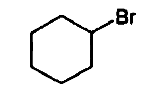 | 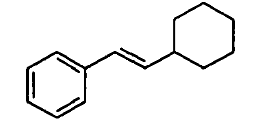 | 35% |

### Beispiel 49

Analog zur Versuchsvorschrift aus Beispiel 36-44 gelingt auch die Umsetzung von Arylbromiden mit Alkenylbromiden.

| Beispiel | R-X | R'-Y | R-R' | Ausbeute |
|---|---|---|---|---|
| 49 | 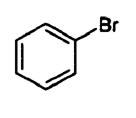 | 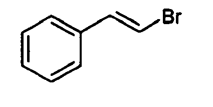 | 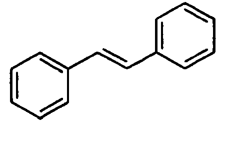 | 19% |

### Beispiel 50 bis 55

Analog zur Versuchsvorschrift aus Beispiel 36-44, wurde 4-Bromtoluol mit Bromcyclohexan umgesetzt. Anstelle von *N,N,N',N'*-Tetramethyl-1,2-diaminocyclohexan (Me₄DACH) wurden elektronisch und sterisch unterschiedliche stickstoffhaltige Additive verwendet.

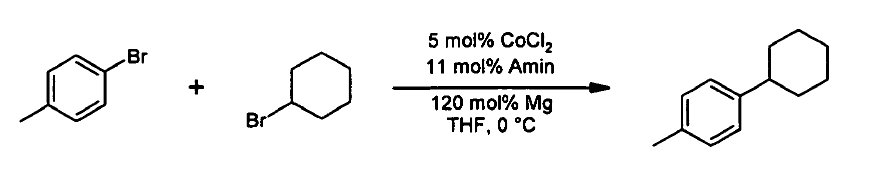

| Beispiel | Additiv | Ausbeute |
|---|---|---|
| 49 | TMEDA | 64 % |
| 50 | DABCO | 30 % |
| 51 | Triethylamin | 3 % |
| 52 | Pyridin | 47 % |
| 53 | 1,10-Phenanthrolin | 10 % |
| 54 | Kein | 6 % |

### Beispiel 55

Analog zur Versuchsvorschrift aus Beispiel 36-44, wurde 2-Bromanisol mit (3-Brombutyl)benzol umgesetzt. Anstelle von *N,N,N',N*'-Tetramethyl-1,2-diaminocyclohexan (Me₄DACH) in racemischer Form wurde die optisch reine 1*R*,2*R*-Form verwendet. Dadurch gelingt die Synthese von 2-(1-Methyl-3-phenyl)propylanisol in stereoisomerenangereicherter Form.

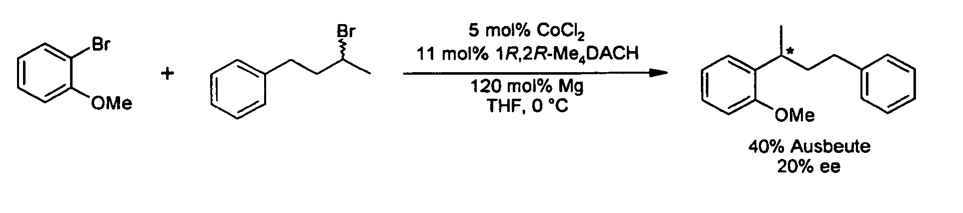

## Patentansprüche

1. Verfahren zur Herstellung organischer Verbindungen der allgemeinen Formel (I)
R-R' (I),
worin
R für einen gegebenenfalls substituierten aromatischen, heteroaromatischen, cycloalkenyl ischen oder alkenylischen Rest steht, und
R' für einen gegebenenfalls substituierten alkylischen, alkenylischen, cycloalkylischen oder cycloalkenylischen Rest steht,
durch Umsetzung einer entsprechenden Verbindung der allgemeinen Formel (II)
R-X (II),
worin
X für Chlor, Brom, Iod, Diazonium, Mesylat (Methansulfonat), Tosylat (*p-* Toluolsulfonat) oder Triflat (Trifluormethansulfonat) steht, und
R die für Formel (I) angegebene Bedeutung hat,
mit einer entsprechenden Verbindung der allgemeinen Formel (III)
R'-Y (III)
worin
Y für Chlor, Brom oder Iod steht, und
R' die für Formel (I) angegebene Bedeutung hat,
**dadurch gekennzeichnet, dass** man die Reaktion in Gegenwart
a) stöchiometrischer Mengen elementaren Magnesiums, bezogen auf die Verbindung der allgemeinen Formel (II) und
b) katalytischer Mengen einer Übergangsmetallverbindung, bezogen auf die Verbindung der allgemeinen Formel (II),wobei die Übergangsmetallverbindung eine Eisenverbindung aus der Gruppe Eisen(II)chlorid, Eisen(III)chlorid, Eisen(II)acetylacetonat Eisen(III)acetylacetonat, Eisen(II)acetat, Eisen(III)acetat, Eisen(II)bromid, Eisen(III)bromid, Eisencarbonyl-Komplex oder Kobalt(II)chlorid oder Kobalt(II)bromid ist,
sowie gegebenenfalls in Gegenwart
c) eines stickstoff-, sauerstoff- und/oder phosphorhaltigen Additivs in katalytischer oder stöchiometrischer Menge bezogen auf die Verbindung der allgemeinen Formel (II), und die Reaktion als Eintopfverfahren durchgeführt wird, bei der die *in situ* als Intermediat entstehende Organomagnesiumverbindung (Grignard-Verbindung) nicht isoliert wird.

2. Verfahren nach Anspruch 1, **dadurch gekennzeichnet, dass** R ein Phenyl-, Naphthyl-, Tolyl-, Anisol-, Kresol-, Anilin-, Benzoesäure-, Pyridin-, 2-Methylpropenyl-, 2-Phenylethylen- oder 1-Cyclohexenylrest ist.

3. Verfahren nach Anspruch 1, **dadurch gekennzeichnet, dass** R' ein C₃-C₁₂-Alkyl-, Cycloalkyl-, C₃-C₁₂-Olefin- oder 1-Cyclohexenylrest ist.

4. Verfahren nach Anspruch 1, **dadurch gekennzeichnet, dass** der alkenylische, cycloakenylische, aromatische oder heteroaromatische Rest R bis zu acht Substituenten tragen kann, welche unabhängig voneinander (C₁-C₁₂)-Alkyl, (C₁-C₁₂)-Cycloalkyl, (C₁-C₁₂)-Alkenyl, (C₁-C₁₂)-Cycloalkenyl, (C₁-C₁₂)-Alkinyl, (C₁-C₁₂)-Aryl, O-[(C₁-C₁₂)-Alkenyl], O-[(C₁-C₁₂)-Aryl), O-Si[(C₁-C₁₂)-Alkyl]ₙ[C₁-C₁₂)Aryl]₃₋ₙ, OC(O)-[(C₁-C₁₂)-Alkyl], OC(O)-[(C₁-C₁₂)-Aryl], NH₂, NH[(C₁-C₁₂)-Alkyl), N[(C₁-C₁₂)-Alkyl]₂, NH[(C₁-C₁₂)-Aryl], N[(C₁-C₁₂)-Aryl]₂, NHC(O)-[(C₁-C₁₂)-Alkyl], N[(C₁-C₁₂)-Alkyl]C(O)-[(C₁-C₁₂)-Alkyl], NHC(O)-[(C₁-C₁₂)-Aryl], N[(C₁-C₁₂)-Alkyl]C(O)-[(C₁-C₁₂)-Aryl], NO₂, NO, S-[(C₁-C₁₂)-Aryl], S-[(C₁-C₁₂)-Alkyl], Fluor, Chlor, Brom, CF₃, CN, COOM, COO-[(C₁-C₁₂)-Alkyl], COO-[(C₁-C₁₂)-Aryl], C(O)NH-[(C₁-C₁₂)-Alkyl], C(O)NH-[(C₁-C₁₂)-Aryl], C(O)N-[(C₁-C₁₂)-Alkyl]₂, C(O)N-[(C₁-C₁₂)-Aryl]₂, CHO, SO₂-[(C₁-C₁₂)-Alkyl), SO-[(C₁-C₁₂)-Alkyl], SO₂-[(C₁-C₁₂)-Aryl), SO-[(C₁-C₁₂)-Aryl), OSO₂(C₁-C₁₂)-Alkyl], OSO₂-[(C₁-C₁₂)-Aryl], PO-[(C₁-C₁₂)-Alkyl]₂, PO-[(C₁-C₁₂)-Aryl]₂, SO₃M, SO₃-[(C₁-C₁₂)-Alkyl], SO₃-[(C₁-C₁₂)-Aryl] oder Si[(C₁-C₁₂)-Alkyl]ₙ[C₁-C₁₂)-Aryl]₃₋ₙ bedeuten, wobei M für ein Alkali- oder Erdalkaliatom und n für eine natürliche Zahl im Bereich von 0 bis 3 steht.

5. Verfahren nach Anspruch 1, **dadurch gekennzeichnet, dass** der alkylische, alkenylische, cycloalkylische oder cycloalkenylische Rest R' gegebenenfalls ein oder mehrere Substituenten tragen kann, welche unabhängig voneinander (C₁-C₁₂)-Alkyl, (C₁-C₁₂)-Cyloalkyl, (C₁-C₁₂)-Alkenyl, (C₁-C₁₂)-Cycloalkenyl, (C₁-C₁₂)-Alkinyl, (C₁-C₁₂)-Aryl, O-[(C₁-C₁₂)-Alkyl), O-[(C₁-C₁₂)-Aryl], O-Si[(C₁-C₁₂)-Alkyl]ₙ[C₁-C₁₂)-Aryl]₃₋ₙ OC(O)-[(C₁-C₁₂)-Alkyl], OC(O)-[(C₁-C₁₂)-Aryl], NH₂, NH[(C₁-C₁₂)-Alkyl], N[(C₁-C₁₂)-Alkyl]₂, NH[(C₁-C₁₂)-Aryl], N[(C₁-C₁₂)-Aryl]₂, NHC(O)-[(C₁-C₁₂)-Alkyl), N[(C₁-C₁₂)-Alkyl)C(O)-[(C₁-C₁₂)-Alkyl), NHC(O)-[(C₁-C₁₂)-Aryl], N[(C₁-C₁₂)-Alkyl]C(O)-[(C₁-C₁₂)-Aryl], NO₂, NO, S-[(C₁-C₁₂)-Aryl], S-[(C₁-C₁₂)-Alkyl], Fluor, Chlor, Brom, CF₃, CN, COOM, COO-[(C₁-C₁₂)-Alkyl], COO-[(C₁-C₁₂)-Aryl], C(O)NH-[(C₁-C₁₂)-Alkyl], C(O)NH-[(C₁-C₁₂)-Aryl], C(O)N-[(C₁-C₁₂)-Alkyl]₂, C(O)N-[(C₁-C₁₂)Aryl]₂, CHO, SO₂-[(C₁-C₁₂)-Alkyl], SO-[(C₁-C₁₂)-Alkyl], SO₂-[(C₁-C₁₂)-Aryl], SO-[(C₁-C₁₂)-Aryl], OSO₂-[(C₁-C₁₂)-Alkyl], OSO₂-[(C₁-C₁₂)-Aryl], PO-[(C₁-C₁₂)-Alkyl]₂, PO-[(C₁-C₁₂)-Aryl]₂, SO₃M, SO₃-[(C₁-C₁₂)-Alkyl], SO₃-[(C₁-C₁₂)-Aryl] oder Si[(C₁-C₁₂)-Alkyl]ₙ[C₁-C₁₂)-Aryl]₃₋ₙ bedeuten, wobei M für ein Alkali- oder Erdalkaliatom und n für eine natürliche Zahl im Bereich von 0 bis 3 steht.

6. Verfahren nach Anspruch 1, **dadurch gekennzeichnet, dass** die Übergangsmetallverbindung in einer Menge von 0,01 bis 50 mol%, bezogen auf die Verbindung der allgemeinen Formel (II), eingesetzt wird.

7. Verfahren nach Anspruch 1, **dadurch gekennzeichnet, dass** als gegebenenfalls zugesetztes stickstoff-, sauerstoff- und/oder phosphorhaltiges Additiv mit einem oder mehreren Stickstoff-, Sauerstoff- und/oder Phosphoratomen gegebenenfalls substituierte Alkylamine, *N*-haltige Heterocyclen, Alkylamide, cyclische Alkylamide, Cycloalkylamine, Cycloalkyldiamine, Alkylimine, Cycloalkylimine, Anilin, Anilin-Derivate, stickstoffhaltige Heteroaromaten, Dialkylether, Alkylarylether, Diarylether, cyclische Ether, Oligoether, Polyether, Triarylphosphane, Trialkylphosphane, Aryldialkylphosphane, Alkyldiarylphosphane und verbrückte Bisphosphane eingesetzt werden.

8. Verfahren nach der Anspruch 1, **dadurch gekennzeichnet, dass** das stickstoff-, sauerstoff- und/oder phosphorhaltige Additiv bevorzugt in einer Menge von 0 bis 200 mol%, bezogen auf die Verbindung der allgemeinen Formel (II), eingesetzt wird.

## Claims

1. Process for preparing organic compounds of the general formula (I)
R-R' (I),
where
R is a substituted or unsubstituted aromatic, heteroaromatic, cycloalkenylic or alkenylic radical and
R' is a substituted or unsubstituted alkylic, alkenylic, cycloalkylic or cycloalkenylic radical,
by reacting a corresponding compound of the general formula (II)
R-X (II),
where
X is chlorine, bromine, iodine, diazonium, mesylate (methanesulphonate), tosylate (p-toluenesulphonate) or triflate (trifluoromethanesulphonate) and
R is as defined for formula (I),
with a corresponding compound of the general formula (III)
R'-Y (III),
where
Y is chlorine, bromine or iodine and
R' is as defined for formula (I),
**characterized in that** the reaction is carried out in the presence of
a) stoichiometric amounts of elemental magnesium, based on the compound of the general formula (II), and
b) catalytic amounts of a transition metal compound, based on the compound of the general formula (II), where the transition metal compound is an iron compound selected from the group consisting of iron(II) chloride, iron(III) chloride, iron(II) acetylacetonate, iron(III) acetylacetonate, iron(II) acetate, iron(III) acetate, iron(II) bromide, iron(III) bromide, iron carbonyl complex or cobalt(II) chloride or cobalt(II) bromide,
and, if appropriate,
c) in the presence of a nitrogen-, oxygen- and/or phosphorus-containing additive in a catalytic or stoichiometric amount, based on the compound of the general formula (II), and the reaction is carried out as a one-pot process in which the organomagnesium compound (Grignard compound) formed *in situ* as intermediate is not isolated.

2. Process according to Claim 1, **characterized in that** R is a phenyl, naphthyl, tolyl, anisole, cresol, aniline, benzoic acid, pyridine, 2-methylpropenyl, 2-phenylethylene or 1-cyclohexenyl radical.

3. Process according to Claim 1, **characterized in that** R' is a C₃-C₁₂-alkyl, cycloalkyl, C₃-C₁₂-olefin or 1-cyclohexenyl radical.

4. Process according to Claim 1, **characterized in that** the alkenylic, cycloalkenylic, aromatic or heteroaromatic radical R can bear up to eight substituents which can be, independently of one another, (C₁-C₁₂) -alkyl, (C₁-C₁₂) -cycloalkyl, (C₁-C₁₂)-alkenyl, (C₁-C₁₂)-cycloalkenyl, (C₁-C₁₂)-alkynyl, (C₁-C₁₂)-aryl, O-[ (C₁-C₁₂)-alkyl], O-[(C₁-C₁₂)-aryl], O-si[(C₁-C₁₂)-alkyl]ₙ[C₁-C₁₂)-aryl3-n, OC(O)-[(C₁-C₁₂)-alkyl], OC(O)-[(C₁-C₁₂)-aryl], NH₂, NH[(C₁-C₁₂)-alkyl], N(C₁-C₁₂)-alkyl]₂, NH[(C₁-C₁₂)-aryl], N[(C₁-C₁₂)-aryl]₂, NHC(O)-[(C₁-C₁₂)-alkyl], N[(C₁-C₁₂)-alkyl]C(O)-[(C₁-C₁₂)-alkyl], NHC(O)-[(C₁-C₁₂) -aryl], N[(C₁-C₁₂)-alkyl]C(O)-[(C₁-C₁₂)-aryl], NO₂, NO, S-[(C₁-C₁₂)-aryl], S-[(C₁-C₁₂) -alkyl], fluorine, chlorine, bromine, CF₃, CN, COOM, COO-[(C₁-C₁₂)-alkyl] , COO-[(C₁-C₁₂)-aryl] , C(O)NH-[(C₁-C₁₂)-alkyl], C(O)NH-[(C₁-C₁₂)-aryl],C(O)N-[(C₁-C₁₂)-alkyl]₂, C(O)N-[(C₁-C₁₂)-aryl]₂,CHO,SO₂-[(C₁-C₁₂)-alkyl], SO-[(C₁-C₁₂)-alkyl],SO₂-[(C₁-C₁₂)-aryl],SO-[(C₁-C₁₂)-aryl], OSO₂-[(C₁-C₁₂)-alkyl], OSO₂-(C₁-C₁₂)-aryl], PO-[(C₁-C₁₂)-alkyl]₂, PO-[(C₁-C₁₂)-acryl]2, SO₃M, SO₃-[(C₁-C₁₂)-alkyl],SO₃-[(C₁-C₁₂)-aryl] orSi[(C₁-C₁₂)-alkyl]ₙ[C₁-C₁₂)-aryl]₃₋ₙ, where M is an alkali metal or alkaline earth metal atom and n is a natural number in the range from 0 to 3.

5. Process according to Claim 1, **characterized in that** the alkylic, alkenylic, cycloalkylic or cycloalkenylic radical R' may, if appropriate, bear one or more substituents which can be, independently of one another, (C₁-C₁₂))-alkyl, (C₁-C₁₂) -cycloalkyl, (C₁-C₁₂)-alkenyl, (C₁-C₁₂)-cycloalkenyl, (C₁-C₁₂) -alkynyl, (C₁-C₁₂) -aryl, O-[(C₁-C₁₂)-alkyl], O-[(C₁-C₁₂)-aryl], O-Si[(C₁-C₁₂)-alkyl]ₙ [C₁-C₁₂)-aryl]₃₋ₙ, OC(O)-[ (C₁-C₁₂) -alkyl], OC(O)-[(C₁-C₁₂)-aryl], NH₂, NH[(C₁-C₁₂)-alkyl], N [(C₁-C₁₂)-alkyl]₂, NH [(C₁-C₁₂)-aryl], N(C₁-C₁₂)-aryl]₂, NHC(O)-[(C₁-C₁₂)-alkyl], N[(C₁-C₁₂)-alkyl]C(O)-[(C₁-C₁₂)-alkyl],NHC(O)-[(C₁-C₁₂)-aryl], N[(C₁-C₁₂)-alkyl]C(O)-[(C₁-C₁₂)-aryl], NO₂, NO, S-[(C₁-C₁₂) -aryl], S-[(C₁-C₁₂)-alkyl], fluorine, chlorine, bromine, CF₃, CN, COOM, COO-[(C₁-C₁₂)-alkyl] , COO-[(C₁-C₁₂)-aryl], C(O)NH-[(C₁-C₁₂)-alkyl], C (O) NH- [(C₁-C₁₂)-aryl], C(O)N-(C₁-C₁₂)-alkyl]₂, C(O)N-[(C₁-C₁₂)-aryl]₂, CHO, SO₂-(C₁-C₁₂)-alkyl], SO-[(C₁-C₁₂)-alkyl],SO₂-[(C₁-C₁₂)-aryl], SO-[(C₁-C₁₂)-aryl], OSO₂-[(C₁-C₁₂)-alkyl], OSO₂-[(C₁-C₁₂)-aryl], PO-[(C₁-C₁₂)-alkyl]₂, PO-[(C₁-C₁₂)-acryl]₂, SO₃M, SO₃-[(C₁-C₁₂)-alkyl], SO₃-[(C₁-C₁₂)-aryl] or Si[(C₁-C₁₂)-alkyl]ₙ[C₁-C₁₂)-aryl]₃₋ₙ, where M is an alkali metal or alkaline earth metal atom and n is a natural number in the range from 0 to 3.

6. Process according to Claim 1, **characterized in that** the transition metal compound is used in an amount of from 0.01 to 50 mol%, based on the compound of the general formula (II).

7. Process according to Claim 1, **characterized in that** substituted or unsubstituted alkylamines, N-containing heterocycles, alkylamides, cyclic alkylamides, cycloalkylamines, cycloalkyldiamines, alkylimines, cycloalkylimines, aniline, aniline derivatives, nitrogen-containing heteroaromatics, dialkyl ethers, alkyl aryl ethers, diaryl ethers, cyclic ethers, oligoethers, polyethers, triarylphosphanes, trialkylphosphanes, aryldialkylphosphanes, alkyldiarylphosphanes and bridged bisphosphanes are used as a nitrogen-, oxygen- and/or phosphorus-containing additive having one or more nitrogen, oxygen and/or phosphorus atoms which may be added.

8. Process according to Claim 1, **characterized in that** the nitrogen-, oxygen- and/or phosphorus-containing additive is preferably used in an amount of from 0 to 200 mol%, based on the compound of the general formula (II).

## Revendications

1. Procédé pour la préparation de composés organiques de formule générale (I),
R-R' (I),
où
R représente un radical aromatique, hétéroaromatique, cycloalcénylique ou alcénylique le cas échéant substitué, et
R' représente un radical alkylique, alcénylique, cycloalkylique ou cycloalcénylique, le cas échéant substitué,
par transformation d'un composé correspondant de formule générale (II)
R-X (II),
où
X représente chlore, brome, iode, diazonium, mésylate (méthanesulfonate), tosylate (p- toluènesulfonate) ou triflate (trifluorométhanesulfonate), et
R a la signification indiquée pour la formule (I),
avec un composé correspondant de formule générale (III)
R'-Y (III),
où
Y représente chlore, brome ou iode, et
R' a la signification indiquée pour la formule (I),
**caractérisés en ce qu'**on réalise la réaction en présence
a) de quantités stoechiométriques de magnésium élémentaire, par rapport au composé de formule générale (II) et
b) de quantités catalytiques d'un composé de métal de transition, par rapport au composé de formule générale (II), le composé de métal de transition étant un composé du fer du groupe formé par le chlorure de fer (II), le chlorure de fer (III), l'acétylacétonate de fer (II), l'acétylacétonate de fer (III), l'acétate de fer (II), l'acétate de fer (III), le bromure de fer (II), le bromure de fer (III), le complexe fer-carbonyle ou le chlorure de cobalt (II) ou le bromure de cobalt (II),
ainsi que le cas échéant en présence
c) d'un additif contenant de l'azote, de l'oxygène et/ou du phosphore en quantité catalytique ou stoechiométrique par rapport au composé de formule générale (II), et la réaction est réalisée sous forme de procédé dans un pot, le composé organique du magnésium formé in situ comme intermédiaire (composé de Grignard) n'étant pas isolé.

2. Procédé selon la revendication 1, **caractérisé en ce que** R est un radical phényle, naphtyle, toluyle, anisol, crésol, aniline, acide benzoïque, pyridine, 2-méthylpropényle, 2-phényléthylène ou 1-cyclohexényle.

3. Procédé selon la revendication 1, **caractérisé en ce que** R' est un radical C₃-C₁₂-alkyle, cycloalkyle, C₃-C₁₂-oléfine ou 1-cyclohexényle.

4. Procédé selon la revendication 1, **caractérisé en ce que** le radical alcénylique, cycloalcénylique, aromatique ou hétéroaromatique R peut porter jusqu'à huit substituants, qui signifient, indépendamment l'un de l'autre, (C₁-C₁₂) -alkyle, (C₁-C₁₂) -cycloalkyle, (C₁-C₁₂) -alcényle, (C₁-C₁₂) -cycloalcényle, (C₁-C₁₂) -alcynyle, (C₁-C₁₂)-aryle, O-[(C₁-C₁₂)-alkyle], O-[(C₁-C₁₂)-aryle], O-Si[(C₁-C₁₂)-alkyl]ₙ[(C₁-C₁₂)-aryle]₃₋ₙ, OC(O)-(C₁-C₁₂)-alkyle], OC(O)-[(C₁-C₁₂)-aryle], NH₂, NH[(C₁-C₁₂)-alkyle], N[(C₁-C₁₂)-alkyle]₂, NH[(C₁-C₁₂)-aryle], N[(C₁-C₁₂)-aryle]₂, NHC(O)-[(C₁-C₁₂)-alkyle], N[(C₁-C₁₂)-alkyl]C(O)-[(C₁-C₁₂)-alkyle], NHC(O)-[(C₁-C₁₂)-aryle], N(C₁-C₁₂)-alkyl]C(O)-[(C₁-C₁₂)-aryle], NO₂, NO, S-[(C₁-C₁₂)-aryle], S-[(C₁-C₁₂) -alkyle], fluor, chlore, brome, CF₃, CN, COOM, COO-[(C₁-C₁₂)-alkyle] , COO-[(C₁-C₁₂)-aryle] , C (O) NH- [(C₁-C₁₂) -alkyle] , C(O)NH- [(C₁-C₁₂)-aryle], C(O)N-[(C₁-C₁₂)-alkyle]₂, C(O)N-[(C₁-C₁₂)-aryle]₂, CHO, SO₂-[(C₁-C₁₂)-alkyle], SO-[(C₁-C₁₂) -alkyle] , SO₂-[(C₁-C₁₂)-aryle], SO-[(C₁-C₁₂)-aryle], OSO₂-[(C₁-C₁₂)-alkyle], OSO₂-[(C₁-C₁₂)-aryle], PO-[(C₁-C₁₂)-alkyle]₂, PO-[(C₁-C₁₂)-aryle]₂, SO₃M, SO₃-[(C₁-C₁₂)-alkyle], SO₃-[(C₁-C₁₂)-aryle] ou Si[(C₁-C₁₂)-alkyl]ₙ[(C₁-C₁₂)-aryle]₃₋ₙ, M représentant un atome de métal alcalin ou alcalino-terreux et n valant un nombre naturel dans la plage de 0 à 3.

5. Procédé selon la revendication 1, **caractérisé en ce que** le radical alkylique, alcénylique, cycloalkylique ou cycloalcénylique R' peut le cas échéant porter un ou plusieurs substituants, qui signifient, indépendamment l'un de l'autre (C₁-C₁₂)-alkyle, (C₁-C₁₂)-cycloalkyle, (C₁-C₁₂)-alcényle, (C₁-C₁₂)-cycloalcényle, (C₁-C₁₂)-alcynyle, (C₁-C₁₂)-aryle, O-[(C₁-C₁₂) -alkyle], O-[(C₁-C₁₂)-aryle], O-Si[(C₁-C₁₂)-alkyl]ₙ[(C₁-C₁₂)-aryle]₃₋ₙ, OC(O)-[(C₁-C₁₂)-alkyle], OC(O)-[(C₁-C₁₂) -aryle], NH₂, NH [(C₁-C₁₂) -alkyle], N[(C₁-C₁₂) -alkyle]₂, NH [(C₁-C₁₂)-aryle], N[(C₁-C₁₂)-aryle]₂, NHC(O)-[(C₁-C₁₂) -alkyle], N[(C₁-C₁₂)-alkyl]C(O)-[(C₁-C₁₂) - alkyle], NHC(O)-[(C₁-C₁₂) -aryle], N[(C₁-C₁₂)-alkyl]C(O)-[(C₁-C₁₂)-aryle], NO₂, NO, S-[(C₁-C₁₂)-aryle], S-[(C₁-C₁₂)-alkyle] , fluor, chlore, brome, CF₃, CN, COOM, COO-[(C₁-C₁₂) -alkyle], COO-[(C₁-C₁₂) -aryle], C(O)NH-[(C₁-C₁₂)-alkyle], C(O)NH-[(C₁-C₁₂)-aryle], C(O)N-[(C₁-C₁₂)-allyle]₂, C(O) N-[(C₁-C₁₂)-aryle]₂, CHO, SO₂-(C₁-C₁₂)-alkyle], SO- [(C₁-C₁₂)-alkyle], SO₂-[(C₁-C₁₂) -aryle], SO-[(C₁-C₁₂)-aryle], OSO₂-[(C₁-C₁₂)-alkyle], OSO₂-[(C₁-C₁₂)-aryle], PO-[(C₁-C₁₂)-alkyle]₂, PO-[(C₁-C₁₂)-aryle]₂, SO₃M, SO₃-[(C₁-C₁₂)-alkyle], SO₃-(C₁-C₁₂)-aryle] ou Si[(C₁-C₁₂)-alkyl]ₙ [(C₁-C₁₂)-aryle]₃₋ₙ, M représentant un atome de métal alcalin ou alcalino-terreux et n valant un nombre naturel dans la plage de 0 à 3.

6. Procédé selon la revendication 1, **caractérisé en ce que** le composé de métal de transition est utilisé en une quantité de 0,01 à 50% en mole, par rapport au composé de formule générale (II).

7. Procédé selon la revendication 1, **caractérisé en ce qu'**on utilise comme additif contenant de l'azote, de l'oxygène et/ou du phosphore, comprenant un ou plusieurs atomes d'azote, d'oxygène et/ou de phosphore, le cas échéant ajouté, des alkylamines, des hétérocycles contenant N, des alkylamides, des alkylamides cycliques, des cycloalkylamines, des cycloalkyldiamines, des alkylimines, des cycloalkylimines, l'aniline, des dérivés d'aniline, des hétéroaromatiques contenant de l'azote, des dialkyléther, des alkylaryléthers, des diaryléthers, des éthers cycliques, des oligoéthers, des polyéthers, des triarylphosphanes, des trialkylphosphanes, des aryldialkylphosphanes, des alkyldiarylphosphanes et des bisphosphanes pontés, le cas échéant substitué(e)s.

8. Procédé selon la revendication 1, **caractérisé en ce que** l'additif contenant de l'azote, de l'oxygène et/ou du phosphore est de préférence utilisé en une quantité de 0 à 200% en mole, par rapport au composé de formule générale (II).
